# EUROPEAN PATENT SPECIFICATION

(11) **EP 0 753 301 B1**
(45) Date of publication and mention of the grant of the patent: **07.05.2003**
(21) Application number: 96115146.1
(22) Date of filing: 07.06.1994
(51) Int. Cl.: A61K 31/4184, A61K 31/549, A61P 9/12

(54) **Combination of a benzimidazole having angiotensin-II antagonistic activity with a diuretic**
Kombination eines Angiotensin-II antagonistisch wirkendes Benzimidazols mit ein Diuretikum
Combinaison d'un benzimidazole ayant un activité antagoniste de l'angiotensine-II avec un diurétique

(30) Priority: 07.06.1993 JP 13552493
(43) Date of publication of application: 15.01.1997
(62) Divisional of application: 94108687.8
(73) Proprietor: TAKEDA CHEMICAL INDUSTRIES, LTD., Chuo-ku, Osaka 541 (JP)
(72) Inventor: Inada, Yoshiyuki, Kawanishi, Hyogo 666 (JP); Kubo, Keiji, Minoo, Osaka 562 (JP)
(74) Representative: Keller, Günter, Dr.

(56) References cited:
- EP-A- 0 400 835
- EP-A- 0 459 136
- EP-A- 0 501 892
- GB-A- 2 270 841
- EUR. J. PHARMACOL., vol. 235, no. 2-3, April 1993, pages 313--316, XP002018430 K. KINUGAWA: "Effects of a nonpeptide angiotensin II receptor antagonist (CV-11974) on [Ca2+]i and cell motion in cultured ventricular myocites."
- WADA T. ET AL.: 'Combined Effects of the Angiotensin II Antagonist Candesartan Cilexetil (TCV-116) and Other Classes of Antihypertensive Drugs in Spontaneously Hypertensive Rats' HYPERTENS.RES, vol. 19, no. 4, December 1996, pages 247 - 254

## Description

### Field of the invention

This invention relates to a pharmaceutical composition for a angiotensin II-mediated disease, namely hypertension, which comprises a compound having angiotensin II antagonistic activity, namely (±)-1-(cyclohexyloxycarbonyloxy)ethyl 2-ethoxy-1-[[2'-(1H-tetrazol-5-yl)biphenyl-4-yl]methyl]-1H-benzimidazole-7-carboxylate] or a salt thereof in combination with a compound having diuretic activity, namely hydrochlorothiazide.

### Background of the invention

Diuretic drugs, due to their having mild hypotensive effects, have long been clinically used as antihypertensive agents. However, as undesirable side effects caused by the use for a long time, influences on metabolism, for example, hypokalemia, hyperuricemia, hyperlipemia and diabetes melitus, have been taken up.

On the other hand, it is disclosed in EP-0425921, EP-0459136 and EP-0520423 that benzimidazole derivatives have angiotensin II antagonistic activities and are useful for the therapy of circulatory diseases including hypertension, cardiac diseases (cardiac insufficiency, myocardial infarction, etc.), cerebral apoplexy, nephritis and arteriosclerosis. The mechanism of the action is considered that the benzimidazole derivatives inhibit the binding of angiotensin II having a strong vasoconstrictive action to an angiotensin II acceptor.

In EP-A-0 400 835 and JPA HS(1993)-132467, it is disclosed that an imidazole derivative having angiotensin II antagonistic action is administered together with a diuretic agent or a calcium antagonistic agent.

### Object of the invention

The invention is intended, by combination of a compound having angiotensin II antagonistic action or a salt thereof with a compound having diuretic action, to perform especially remarkable effects, to reduce undesirable side effects and to cover up defects observed in administration of a medicine consisting of a single component.

### Summary of the invention

Circumstances being such as above, the present inventors have made extensive and intensive studies on the effects of co-use of a benzimidazole derivative having angiotensin antagonistic activity with a compound having diuretic activity, and, as a result, they have found that the co-use performs especially remarkable effects which were not observed in the administration of the respective compounds singly, thus accomplishing the present invention.

More specifically, the present invention relates to a pharmaceutical composition for the treatment of hypertension, which comprises (±)-1-(cyclohexyloxycarbonyloxy)ethyl 2-ethoxy-1-[[2'-(1H-tetrazol-5-yl)biphenyl-4-yl]methyl]-1H-benzimidazole-7-carboxylate (formula (I)) or a pharmaceutically acceptable salt thereof in combination with hydrochlorothiazide.

As the salt of the compound of the formula (I), pharmaceutically acceptable salts are used, e.g., a salt with an inorganic base, organic base, inorganic acid, organic acid, or basic or acidic amino acid. Inorganic bases appropriate to form the salt include alkali metals such as sodium or potassium, alkali earth metals such as calcium and magnesium or aluminum, and ammonia. Organic bases appropriate to form the salt include trimethylamine, triethylamine, pyridine, picoline, ethanolamine, diethanolamine, triethanolamine, dicyclohexylamine, and N,N'-dibenzylethylenediamine. Inorganic acids appropriate to form the salt include hydrochloric acid, hydroboric acid, nitric acid, sulfuric acid, and phosphoric acid. Organic acids appropriate to form the salt include formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Basic amino acids to form the salt include arginine, lysine and ornithine. Acidic amino acids to form the salt include aspartic acid and glutamic acid.

The pharmaceutical composition of the present invention can be administered orally or non-orally in the form of, for example, granules, powders, tablets, capsules, syrup, suppositories, injections, emulsions, elixir, suspensions or solutions, by mixing these effective components, individually or simultaneously, with pharmaceutically acceptable carriers, excipients, binders, diluents or the like. In the case of formulating the effective components individually, while thus individually formulated agents can be administered in the form of their mixture prepared by using, for example, a diluent when administered, the individually formulated agents can also be administered separately or simultaneously or with time intervals to the one and same subject.

The pharmaceutical composition of the present invention can be formulated in accordance with conventional procedures. In the present specification, "non-orally" include subcutaneous injection, intravenous injection, intramuscular injections, intraperitoneal injection or instillation. Injectable preparations, for example, sterile injectable aqueous suspensions or oil suspensions can be prepared by known procedure in the fields concerned, using a suitable dispersant or wetting agent and suspending agent. The sterile injections may be in the state of, for example, a solution or a suspension, which is prepared with a nontoxic diluent administrable non-orally, e.g. an aqueous solution, or with a solvent employable for sterile injection. Examples of usable vehicles or acceptable solvents include water, Ringer's solution and an isotonic aqueous saline solution. Further, a sterile non-volatile oil can usually be employed as solvent or suspending agent.

Any non-volatile oil and a fatty acid can be used for this purpose, which includes natural or synthetic or semi-synthetic fatty acid oil or fatty acid, and natural or synthetic or semi-synthetic mono- or di- or tri-glycerides.

Rectal suppositories can be prepared by mixing the drug with a suitable non-irritable vehicle, for example, cocoa butter and polyethylene glycol, which is in the solid state at ordinary temperatures, in the liquid state at temperatures in intestinal tubes and melts in rectum to release the drug.

As a solid formulation for oral administration, mention is made of powders, granules, tablets, pills and capsules as referred to in the above. In such formulations as exemplified above, the active component compounds can be mixed with at least one additive, for example, sucrose, lactose, cellulose sugar, mannitol,maltitol, dextran, starch, agar, alginates, chitins, chitosans, pectins, tragacanth gum, gum arabic, gelatins, collagens, casein, albumin, synthetic or semi-synthetic polymers or glycerides. These formulations can contain, as in conventional cases, further additives, for example, an inactive diluent, a lubricant such as magnesium stearate, a preservative such as paraben or sorbic acid, an anti-oxidant such as ascorbic acid, α-tocopherol or cysteine, a disintegrator, a binder, a thickening agent, a buffer, a sweetener, a flavoring agent and a perfuming agent. Tablets and pills can further be prepared with enteric 5 coating. Examples of liquid preparations for oral administration include pharmaceutically acceptable emulsions, syrups, elixirs, suspensions and solutions, which may contain an inactive diluent, for example, water, which is conventionally employed in the field concerned.

The pharmaceutical composition of this invention is less toxic, and is used as a medicine for animals, especially mammals (e.g. human being, dog, rabbit, mouse, etc.), which can be advantageously used for a angiotensin II-mediated disease, namely hypertension.

The pharmaceutical composition of this invention is formulated by combining a compound having angiotensin II antagonistic activity or a salt thereof with a compound having diuretic activity. This composition serves to decrease the dosages of the individual effective components, and, as a result, suppresses undesirable side effects observed in the case of administering the respective compounds singly.

The dose of a specific patient is dependent on the age, body weight, general health conditions, sex, diet, dose interval, administration routes, excretion rate, combinations of drugs and conditions of the diseases treated, while taking these and other necessary factors into consideration.

Typical daily doses of the compositions having various combinations of a compound represented by the formula (I) or a salt thereof and a compound having diuretic activity are within the range of from 1/50 of the minimal recommendable clinical dose to maximal recommendable dose in the case of practical administration of these compounds individually.

For example, a compound represented by the formula (I) having an angiotensin II antagonistic activity to be administered at a dose of 0.01 to 150 mg/patient/day can be administered at a dose of 0.0002 to 150 mg/patient/day, preferably 0.001 to 60 mg/patient/day, more preferably 0.01 to 20 mg/patient/day by combining with the following daily doses of the following compound:
hydrochlorothiazide (5 to 200 mg). Needless to say, while these dosage ranges can be adjusted by a necessary unit base for dividing a daily dose, as described above, such doses are decided depending on the diseases to be treated, conditions of such diseases, the age, body weight, general health conditions, sex, diet of the patient then treated, dose intervals, administration routes, excretion rate, and combinations of drugs, while taking these and other necessary factors into consideration.

The desired unit dose of the composition of this invention is administered once or twice daily (preferably once).

For example, the unit dose composition contains 0.0002 to 150 mg, preferably 0.001 to 60 mg, more preferably 0.01 to 20 mg of a compound represented by the formula (I) by combining with the following amount of the following compound:
hydrochlorothiazide (5 to 200 mg). The amount of hydrochlorothiazide present in a dosage unit is from 5 mg to 200 mg, preferably 5 mg to 100 mg, more preferably 5 to 50 mg.

By the following test examples and working examples, the present invention will be illustrated in more detail.

The physiological activities of the composition comprising a compound having angiotensin II antagonistic activity or a salt thereof and a compound having diuretic activity are described by the following test examples.

### Test Example 1

### Antihypertensive activity in spontaneously hypertensive rats (SHR) by the co-administration with a diuretic drug

### Compound 1: (±)-1-(cyclohexyloxycarbonyloxy)ethyl 2-ethoxy-1-[[2'-(1H-tetrazol-5-yl)biphenyl-4-yl]methyl]-1H-benzimidazole-7-carboxylate

### HCT: hydrochlorothiazide

Method: Male SHR of 20 week old were divided into 6 groups (five animals in one group). The respective groups were administered orally with the compound 1 (0.1 or 1 mg/kg,p.o.) or HCT (10 mg/kg, p.o.) alone or both drugs simultaneously once a day for two weeks. On the first, 7th and 14th day at 5 hr after the administration, blood pressure of each test animal was measured by the tail cuff method under unanesthesia. Results: As shown in Table 1. Single dose of HCT (10mg/kg/day, p.o.) did not show antihypertensive action. The compound 1 (0.1 and 1 mg/kg/day) showed dose dependent antihypertensive action. Efficiency of the antihypertensive activity of the compound 1 was enhanced by its co-administration with HCT. The antihypertensive activity observed by the combination of the compound 1 (0.1 mg/kg) and HCT was stronger or substantially the same as that observed by administering the compound 1 alone (1 mg/kg). This result shows that the combination of both drugs can decrease the dosages of the respective drugs.

**Table 1**

| Antihypertensive activity by the combination of compound 1 and the diuretic drug in spontaneously hypertensive rats | | | | |
|---|---|---|---|---|
| Test group (dosage) mg/kg/day, p.o. | Before admin. | 1 day | 1 week | 2 weeks |
| | | (blood pressure: mmHg) | | |
| Control - | 183 ± 2 | 183 ± 1 | 179 ± 2 | 181 ± 4 |
| HCT (10) | 186 ± 3 | 178 ± 3 | 177 ± 3 | 181 ± 2 |
| Cpd. 1 (0.1) | 183 ± 2 | 161 ± 5 | 155 ± 3 | 162 ± 3 |
| Cpd. 1 (1) | 186 ± 2 | 153 ± 5 | 138 ± 2 | 135 ± 3 |
| HCT(10)+Cpd.1 (0.1) | 186 ± 4 | 137 ± 5 | 129 ± 5 | 139 ± 3 |
| HCT(10)+Cpd.1 (1) | 187 ± 2 | 132 ± 3 | 106 ± 5 | 108 ± 4 |
| Numerical values: average values ± standard error | | | | |

As is apparent from this test example , the composition comprising the compound having angiotensin II antagonistic activity or a salt thereof and the compound having diuretic activity enhances the action of the respective drug administered singly and can decrease the dosages of the respective drugs. As a result, suppression of the occurrence of undesirable side effects observed when these drugs are administered singly can be expected to a considerable extent.

### Working Example

### Formulation Examples

The pharmaceutical composition of the present invention, formulated by combination of a compound having angiotensin II antagonistic activity represented by the formula (I) or a salt thereof and a compound having diuretic activity, can be prepared by the prescriptions described as follows.

### 1. Capsules

| | | |
|---|---|---|
| (1) | (±)-1-(cyclohexyloxycarbonyloxy)ethyl 2-ethoxy-1-[[2'-(1H-tetrazol-5-yl)biphenyl-4-yl] methyl]-1H-benzimidazole-7-carboxylate | 1 mg |
| (2) | hydrochlorothiazide | 25 mg |
| (3) | lactose | 64 mg |
| (4) | microcrystalline cellulose | 70 mg |
| (5) | magnesium stearate | 10 mg |
| | one capsule | 180 mg |

(1), (2), (3), (4) and 1/2 of (5) were mixed and then granulated. To the granules was added the remainder of (5), and the whole formulation was filled into a gelatin capsule.

### 2. Tablets

| | | |
|---|---|---|
| (1) | (±)-1-(cyclohexyloxycarbonyloxy)ethyl 2-ethoxy-1-[[2'-(1H-tetrazol-5-yl)biphenyl-4-yl] methyl]-1H-benzimidazole-7-carboxylate | 1 mg |
| (2) | hydrochlorothiazide | 25 mg |
| (3) | lactose | 71.4 mg |
| (4) | corn starch | 20 mg |
| (5) | polyethylene glycol | 2.6 mg |
| (6) | hydroxypropyl cellulose | 4 mg |
| (7) | carmellose calcium | 5.6 mg |
| (8) | magnesium stearate | 0.4 mg |
| | one tablet | 130 mg |

(1), (2), (3), (4), (5), 2/3 of (6), 2/3 of (7) and 1/2 of (8) were mixed and then granulated. To the granules were added the remainders of (6), (7) and (8), followed by subjecting the mixture to compression molding.

### 3 . Capsules

| | | |
|---|---|---|
| (1) | (±)-1-(cyclohexyloxycarbonyloxy)ethyl 2-ethoxy-1-[[2'-(1H-tetrazol-5-yl)biphenyl-4-yl] methyl]-1H-benzimidazole-7-carboxylate | 4 ∼ 12 mg |
| (2) | hydrochlorothiazide | 6.25 mg |
| (3) | lactose | 64 mg |
| (4) | microcrystalline cellulose | 70 mg |
| (5) | magnesium stearate | 10 mg |
| | one capsule | 154.25 ∼ 162.25 mg |

(1), (2), (3), (4) and 1/2 of (5) were mixed and then granulated. To the granules was added the remainder of (5), and the whole was filled into a gelatin capsule.

### 4. Tablets

| | | |
|---|---|---|
| (1) | (±)-1-(cyclohexyloxycarbonyloxy)ethyl 2-ethoxy-1-[[2'-(1H-tetrazol-5-yl)biphenyl-4-yl] methyl]-1H-benzimidazole-7-carboxylate | 4 ∼ 12 mg |
| (2) | hydrochlorothiazide | 6.25 mg |
| (3) | lactose | 71.4 mg |
| (4) | corn starch | 20 mg |
| (5) | polyethylene glycol | 2.6 mg |
| (6) | hydroxypropyl cellulose | 4 mg |
| (7) | carmellose calcium | 5.6 mg |
| (8) | magnesium stearate | 0.4 mg |
| | one tablet | 114.35 ∼ 122.35 mg |

(1), (2), (3), (4), (5), 2/3 of (6), 2/3 of (7) and 1/2 of (8) were mixed and then granulated. To the granules were added the remainders of (6), (7) and (8), followed by subjecting the mixture to compression molding.

### 5. Capsules

| | | |
|---|---|---|
| (1) | (±)-1-(cyclohexyloxycarbonyloxy)ethyl 2-ethoxy-1-[[2'-(1H-tetrazol-5-yl )biphenyl-4-yl] methyl]-1H-benzimidazole-7-carboxylate | 2 mg |
| (2) | hydrochlorothiazide | 6.25 ∼ 25 mg |
| (3) | lactose | 64 mg |
| (4) | microcrystalline cellulose | 70 mg |
| (5) | magnesium stearate | 10 mg |
| | one capsule | 152.25 ∼ 171 mg |

(1), (2), (3), (4) and 1/2 of (5) were mixed and then granulated. To the granules was added the remainder of (5), and the whole was filled into a gelatin capsule.

### 6. Tablets

| | | |
|---|---|---|
| (1) | (±)-1-(cyclohexyloxycarbonyloxy)ethyl 2-ethoxy-1-[[2'-(1H-tetrazol-5-yl)biphenyl-4-yl] methyl]-1H-benzimidazole-7-carboxylate | 2 mg |
| (2) | hydrochlorothiazide | 6.25 ∼ 25 mg |
| (3) | lactose | 71.4 mg |
| (4) | corn starch | 20 mg |
| (5) | polyethylene glycol | 2.6 mg |
| (6) | hydroxypropyl cellulose | 4 mg |
| (7) | carmellose calcium | 5.6 mg |
| (8) | magnesium stearate | 0.4 mg |
| | one tablet | 112.35 ∼ 131.1 mg |

(1), (2), (3), (4), (5), 2/3 of (6), 2/3 of (7) and 1/2 of (8) were mixed and then granulated. To the granules were added the remainders of (6), (7) and (8), followed by subjecting the mixture to compression molding.

## Claims

1. Use of a combination of (±)-1-(cyclohexyloxycarbonyloxy)ethyl 2-ethoxy-1-[[2'-(1H-tetrazol-5-yl)biphenyl-4-yl]methyl]-1H-benzimidazole-7-carboxylate or a pharmaceutically acceptable salt thereof with hydrochlorothiazide for the manufacture of a medicament to be used as a prophylactic or therapeutic drug for hypertension.

2. A pharmaceutical composition for the treatment of hypertension, which comprises (±)-1-(cyclohexyloxycarbonyloxy)ethyl 2-ethoxy-1-[[2'-(1H-tetrazol-5-yl)biphenyl-4-yl]methyl]-1H-benzimidazole-7-carboxylate or a pharmaceutically acceptable salt thereof in combination with hydrochlorothiazide.

## Patentansprüche

1. Verwendung einer Kombination von (±)-1-(Cyclohexyloxycarbonyloxy)ethyl-2-ethoxy-1-[[2'-(1H-tetrazol-5-yl)biphenyl-4-yl]methyl]-1H-benzimidazol-7-carboxylat oder einem pharmazeutisch annehmbaren Salz davon mit Hydrochlorthiazid zur Herstellung eines Arzneimittels, das als prophylaktisches oder therapeutisches Arzneimittel für Bluthochdruck verwendet wird.

2. Pharmazeutische Zusammensetzung zur Behandlung von Bluthochdruck, die (±)-1-(Cyclohexyloxycarbonyloxy)ethyl-2-ethoxy-1-[[2'-(1H-tetrazol-5-yl)biphenyl-4-yl]methyl]-1H-benzimidazol-7-carboxylat oder ein pharmazeutisch annehmbares Salz davon in Kombination mit Hydrochlorthiazid enthält.

## Revendications

1. Emploi d'une combinaison de (±)-2-éthoxy-1-[[2'-(1H-tétrazol-5-yl)biphényl-4-yl]méthyl]-1H-benzimidazole-7-carboxylate de 1-(cyclohexyloxycarbonyloxy)éthyle, ou d'un sel pharmacologiquement admissible de ce composé, et d'hydrochlorothiazide dans la fabrication d'un médicament destiné à servir d'agent prophylactique ou thérapeutique contre l'hypertension.

2. Composition pharmaceutique pour traitement de l'hypertension, qui contient du (±)-2-éthoxy-1-[[2'-(1H-tétrazol-5-yl)biphényl-4-yl]méthyl]-1H-benzimidazole-7-carboxylate de 1-(cyclohexyloxycarbonyloxy)éthyle, ou un sel pharmacologiquement admissible de ce composé, combiné avec de l'hydrochlorothiazide.
